(19) Europäisches Patentamt
European Patent Office
Office européen des brevets

(11) **EP 3 296 903 A1**

(12) **EUROPEAN PATENT APPLICATION**

(43) Date of publication:
21.03.2018 Bulletin 2018/12

(51) Int Cl.:
***G06F 19/00*** *(2018.01)*

(21) Application number: **17166977.3**

(22) Date of filing: **19.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.09.2016 DE 102016218005**
**20.09.2016 GB 201615986**

(71) Applicant: **Fujitsu Limited**
**Kawasaki-shi, Kanagawa 211-8588 (JP)**

(72) Inventors:
• **HU, Bo**
**Winchester, Hampshire SO23 7DT (GB)**
• **NASEER BUTT, Aisha**
**Hayes, Middlesex UB4 8LB (GB)**

(74) Representative: **Wilding, Frances Ward**
**Haseltine Lake LLP**
**Lincoln House, 5th Floor**
**300 High Holborn**
**London WC1V 7JH (GB)**

(54) **METHOD AND APPARATUS FOR DISCOVERING A SEQUENCE OF EVENTS FORMING AN EPISODE IN A SET OF MEDICAL RECORDS FROM A PATIENT**

(57) An apparatus for discovering a sequence of events in a set of medical records from a patient, the sequence forming an episode of a medical condition, the apparatus comprising:
a state transition learner, to parse published clinical guidelines and to extract probabilities of transition between a number of states of a medical condition as a state transition model;
a clinical finding learner, to extract typical findings of the medical condition from domain knowledge as a finding model and to compute the probability of a particular finding for a particular state and to save the probabilities in an overall episode model including the state transition model and the finding model; and
an episode grouper, to use the overall episode model, and the set of medical records, and to discover a sequence of events that can be grouped into an episode.

Figure 1

EP 3 296 903 A1

**Description**

The present invention relates to a system and method to detect an episode of a disease or other medical condition, for example by applying machine learning methods. It has applications in the areas of healthcare profiling, healthcare monitoring and improvement, and even as an aid to selection of treatment.

At the onset of certain medical conditions, a patient (or subject) can demonstrate several symptoms and be treated separately or jointly with different treatments. Also, several conditions may jointly affect a patient: this is known as co-morbidity. In this document, a disease/condition episode is defined as including any observable symptoms, payable investigative method, treatments, and medications associated with an instance of that disease/condition.

For instance, a typical measles episode can consist of the following states: infection, incubation, non-specific/mild period, acute period, recovery, and communicable period. At different stages, a patient can demonstrate different symptoms and signs. The patient can be affected by high temperature, cough, headaches, and rash. The patient may be subject to blood tests and chest x-rays. Severe measles can have complications demonstrating other symptoms and signs which need to be dealt with during the patient's interaction with the healthcare system. All these need to be grouped into one episode to enable better healthcare statistics/profiling, improved treatment and also non-medical improvements such better payment management under medical insurance. Also, long term conditions can have acute episode, e.g. a bipolar disorder can have a mania episode, during which period all the interactions should be properly categorised for better patient care.

When treating a patient within one particular episode, the disease progresses from one disease state (with particular symptoms) to another (with different symptoms). For instance, an episode of chest-infection can start with high fever, moving to persistent coughing, moving to asthmatic symptoms, moving to fully recovery or LTC asthma.

These are different states of a particular instance of the disease and may require different medication strategies.

Detecting an episode of a certain disease (for example the start and end of an episode and/or the events that are attributable to that episode) is important in health care in general and in medical insurance industries in particular. The reasons are as follows:

1. In order to have well targeted studies in the medical domain, it is useful to differentiate comorbid diseases. Such diseases can then be studied separately to avoid data "pollution".
2. Certain conditions may be caused by the treatment of prior conditions. Also it is equally important to understand the fluctuation of long term conditions. It is therefore necessary to clearly define and separate different episodes.
3. Medical claims (for example of the same episode) are normally grouped together for a single insurance payout. This also applies to countries providing non-contributory healthcare welfare, e.g. UK, where public budget needs to be carefully studied and defined.

Episode defining or episode grouping is not a trivial task. Thus far, the dominant solution is based on expert opinions. This approach is, however, often prone to errors.

1. Expert opinions only provide ranges of values based on "normal" conditions. For cases falling out the boundaries of such normal ranges, the episode definition is difficult.
2. Human conditions vary from individual to individual and even from time to time with respect to the same individual. The "normal" range cannot cover all the inter- and intra-individual variances.

The inventors have come to the realisation that the inefficiency of current practice calls for a dynamic approach for episode grouping.

According to an embodiment of a first aspect of the invention, there is provided an apparatus for discovering a sequence of events in a set of medical records from a patient, the sequence forming an episode of a medical condition, the apparatus comprising:

a state transition learner, to parse published clinical guidelines and to extract probabilities of transition between a number of states of a medical condition as a state transition model;
a clinical finding learner, to extract typical findings of the medical condition from domain knowledge as a finding model and to compute the probability of a particular finding for a particular state and to save the probabilities in an overall episode model including the state transition model and the finding model; and
an episode grouper, to use the overall episode model, and the set of medical records to discover a sequence of events, to group the sequence of events into an episode of the medical condition, and to differentiate the medical condition from apparently similar medical conditions or co-morbidities, or both.

These features allow discovery of an episode without necessarily involving a human expert and by using different knowledge sources which are publically available. They allow separation of a sequence of events associated with a particular condition from other events, and thus differentiation from other conditions which may be similar and from other symptoms occurring at the same time but for different causes (co-morbidities). The same features can even also allow preliminary identification of a condition, because once a match is made to an episode (or several candidate episodes), it is possible to narrow down the courses of disease development specific to that condition.

An episode is a single (significant) occurrence/instance of a certain illness (which is used synonymously and interchangeably herein with medical condition or disease). It can be one occurrence of a longer series of occurrences. An episode is normally defined as the group of symptoms and signs from the onset of certain relevant symptoms and signs to the disappearance of the symptoms and signs. Each episode can have a number of states. Normally, a "naïve" episode grouper or simple episode grouper will split data to define an episode from the first interaction between a patient and the healthcare system until the patient is deceased or discharged for that condition.

The purpose of an episode grouper according to invention embodiments, and as explained in more detail later herein, can be to identify which findings (e.g. symptoms/signs, treatments and medicines) belong to one episode of certain disease. This can be important information for diagnostic assistance, analysis, insurance and quality assurance purposes.

The term finding(s) or observation(s) is used to refer to observable patient interactions which are thus effectively medical findings. Hence the term can include medical intervention and medication, as well as complaints, symptoms and diagnosis as noted by a healthcare practitioner.

Events are findings coming from a particular patient record which is to be grouped or classified, for example according to a learnt model, as described in more detail later. (An "event" in an individual's medical record is an observable medical result or medical intervention that could have significant meaning in the course of diseases.) So, in short, events are documented individual instances of findings. Findings refer to the general model.

The episode grouper can be to discover a sequence as a subset of events in the set of medical record that can be grouped into an episode, and to exclude remaining events as not forming part of the medical condition. This allows the separation of co-morbidities.

The state transition learner can be arranged to derive the probability of transition between states from internet search results. This may be using internet search results (and, for example, co-occurrence of the states in the search results). The internet search results can be confined to one or more on-line medical publications to give better accuracy.

The clinical learning finder can compute the probability of a particular finding for a particular state in the state transition model, preferably using a training data set, for instance a set of patients' records for the medical condition in question. In this way, each finding from the domain knowledge, such as from on-line medical protocols and guidelines, is mapped to the states in the state transition model based on the occurrence of the finding in each state. Of course if the finding does not occur in a particular state in the medical records (or the level is below a threshold), then the probability can be set to zero, and there is effectively no link between the finding and the state.

Thus the clinical learning finder can compute the probability of a particular finding for a particular state using co-occurrence of the finding and the state in public data.

The overall episode model can contain, for each of one or more medical conditions, links between the findings in the finding model for the medical condition and the states in the state transition model for that medical condition and probabilities associated with the links. Preferably, the model covers a wide range of medical conditions, and holds a separate, individual linked state transition and finding model for each of them.

The overall episode model also preferably contains links to a leak term and probabilities associated with the links. Here, the leak term corresponds to a situation in which a finding is observed which is not relevant to any state in the state transition model for the medical condition.

The episode grouper may match sequences of events in the set of medical records to the overall episode model and detect the best match between a sequence of events and the overall episode model.

For example, the episode grouper is to match the powerset of the sequence of events in the set of medical records to the overall episode model, with the exception of the empty set and/or any sets including a number of events below a threshold. It is important that the events are kept in the correct order in the powerset, but events in the time line may be omitted (and for instance included later in an episode of another condition). The threshold may be 2 events, so that a "sequence" of a single event is not assessed for a match with an episode. The various subsets of sequences can be assessed in any order, for example in random order or fully/partially in parallel.

The episode grouper may match sequences of events to the overall episode model by calculating the probability of arriving at the sequence of events in the set of medical records for each sequence, based on a given initial state in the patient medical records, the state transition model and based on the probability of observable medical findings in the overall episode model corresponding to the events in the sequence of events.

The episode grouper may process remaining events in the set of medical records once a sequence of events has been grouped into an episode, by matching sequences of remaining events. This allows other conditions to be identified on the basis of co-morbidities which might otherwise have been categorised as part of the same condition.

According to an embodiment of a second aspect of the invention, there is provided a method for discovering a sequence of events in a set of medical records from a patient, the sequence forming an episode of a medical condition, the method comprising:

parsing published clinical guidelines and extracting probabilities of transition between a number of states of a medical condition as a state transition model;
extracting typical findings of the medical condition from domain knowledge as a finding model;
computing the probability of a particular finding for a particular state;
saving the probabilities in an overall episode model including the state transition model and the finding model; and
using the overall episode model, and the set of medical records to discover a sequence of events, to group the sequence of events into an episode of the medical condition, and to differentiate the medical condition from apparently similar medical conditions or co-morbidities.

According to an embodiment of a third aspect of the invention, there is provided a computer program which when executed on a computer apparatus carries out a method for discovering a sequence of events in a set of medical records from a patient, the sequence forming an episode of a medical condition, the method comprising:

parsing published clinical guidelines and extracting probabilities of transition between a number of states of a medical condition as a state transition model;
extracting typical findings of the medical condition from domain knowledge as a finding model;
computing the probability of a particular finding for a particular state;
saving the probabilities in an overall episode model including the state transition model and the finding model; and
using the overall episode model, and the set of medical records to discover a sequence of events, to group the sequence of events into an episode of the medical condition, and to differentiate the medical condition from apparently similar medical conditions or co-morbidities.

A method or computer program according to preferred embodiments of the present invention can comprise any combination of the apparatus aspects, but without limitation to specific hardware. Methods or computer programs according to further embodiments can be described as computer-implemented in that they require processing and memory capability.

The apparatus according to preferred embodiments may be described as configured or arranged to, or simply "to" carry out certain functions. This configuration or arrangement could be by use of hardware or middleware or any other suitable system. In preferred embodiments, the configuration or arrangement is by software.

Thus according to one aspect there is provided a program which, when loaded onto at least one computer configures the computer to become the apparatus according to any of the preceding apparatus definitions or any combination thereof.

According to a further aspect there is provided a program which when loaded onto the at least one computer configures the at least one computer to carry out the method steps according to any of the preceding method definitions or any combination thereof.

In general the computer may comprise the elements listed as being configured or arranged to provide the functions defined. For example this computer may include memory, processing, and a network interface.

The invention can be implemented in digital electronic circuitry, or in computer hardware, firmware, software, or in combinations of them. The invention can be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program can be in the form of a stand-alone program, a computer program portion or more than one computer program and can be written in any form of programming language, including compiled or interpreted languages, and it can be deployed in any form, including as a stand-alone program or as a module, component, subroutine, or other unit suitable for use in a data processing environment. A computer program can be deployed to be executed on one module or on multiple modules at one site or distributed across multiple sites and interconnected by a communication network.

Method steps of the invention can be performed by one or more programmable processors executing a computer program to perform functions of the invention by operating on input data and generating output. Apparatus of the invention can be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention can be performed in a different order and still achieve desirable results. Multiple test script versions can be edited and invoked as a unit without using object-oriented programming technology; for example, the elements of a script object can be organized in a structured database or a file system, and the operations described as being performed by the script object can be performed by a test control program.

Elements of the invention have been described using the terms "learner", "grouper" etc. The skilled person will appreciate that such functional terms and their equivalents may refer to parts of the system that are spatially separate but combine to serve the function defined. Equally, the same physical parts of the system may provide two or more of the functions defined.

For example, separately defined means may be implemented using the same memory and/or processor as appropriate.

Preferred features of the present invention will now be described, purely by way of example, with references to the accompanying drawings, in which:-

Figure 1 is a block diagram of main system components in an embodiment of the invention
Figure 2 is a flow chart of a method in a general embodiment;
Figure 3 is a diagram of a generic episode model, constructed from a state transition model and a finding model;
Figure 4 is a diagram of a specific episode model;
Figure 5 is a conceptual diagram which depicts the distribution of computations between a master machine and slave machines;
Figure 6 is a flow chart of model construction;
Figure 7 is a flow chart of episode discovery steps; and
Figure 8 is a diagram of suitable hardware for implementation of invention embodiments.

## Embodiments

In invention embodiments, a framework for episode grouping is introduced. It consists of, among other, the following key modules:

A clinical guideline process modeller and reasoning engine, which acts as a (disease) **state transition learner**: this module can parse published clinical guidelines and mine a process model that covers the "typical" state transition of a medical condition or disease. The purpose of this module is to establish a disease model (or disease progression model) that formalises how a disease transforms between different stages (states) based on the definition of guidelines.

A disease finding modeller, which acts as a **clinical finding learner**: this module extracts typical features of a disease in terms of complaints, symptoms and medications. The primary purpose of this module is to define and discover the relationship between diseases and symptoms/signs and complaints, but it also finds the relationship between these findings and the states in the state transition model.

An episode miner, or **episode grouper**: this module extracts events from a patient record and infers the onset and end of a given disease episode. The purpose of this module is to take the above two knowledge models as well as a patient record and to output a sequence of events that are considered belonging to the same episode.

These modules and their interconnections and external links are detailed in the following sections.

## System components

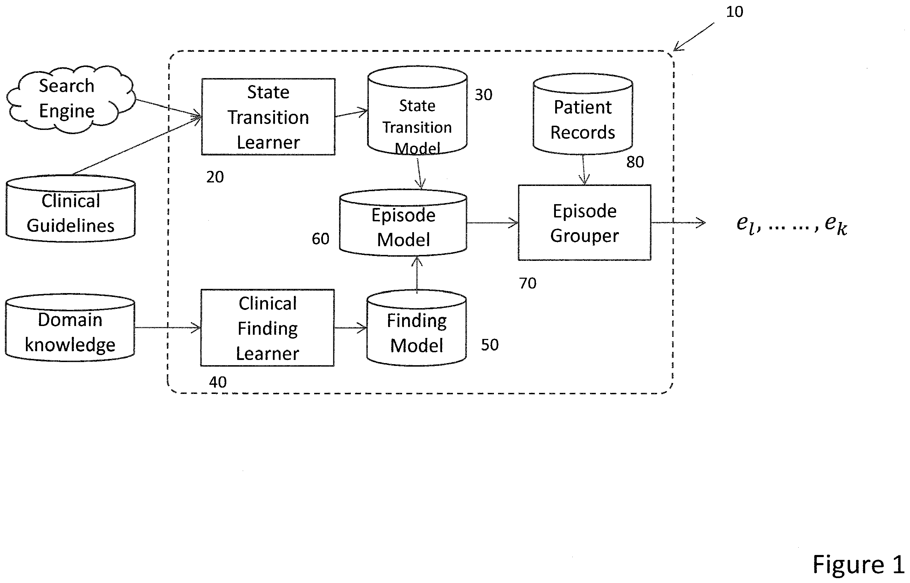

The key components of the system 10 are illustrated in figure 1:

External data sources: the clinical guidelines shown to the left in figure 1 are published guidelines by the authorities to define the course and actions of diseases; the search engine is a normal internet search engine; domain knowledge (bottom left) is the repository for domain expertise, in this case, including medical findings. These are all data sources and accessed by the apparatus/system 10.

The state transition learner 20 is part of the apparatus 10 and leverages the data from the clinical guidelines and search engine to construct a state transition model 30 including the number of states and the probability of transition among different states. This is then stored in a data store.

The clinical finding learner 40 extracts the findings for a medical condition into a **(medical) finding model 50** and computes the probability of observing a particular finding for a given state. These sets of probability are than saved in the **overall episode model** 60. As mentioned above, "finding" is used herein as a general term for medical data associated with the model and refers to all types of clinical findings, such as symptoms, signs, etc. as well as key medical interventions (treatments).

The episode grouper 70 takes the disease state transition model, probability model of medical findings associated with the states and a set of medical records 80 of a patient (for example in a past time window T) and detects the sequence of key findings (treatments/medications/symptoms etc.) that should be grouped into one episode.

The system outputs the grouped sequence of events, $e_l, \dots e_k$. This may be on paper, on screen to a user, as an email or in any other suitable fashion.

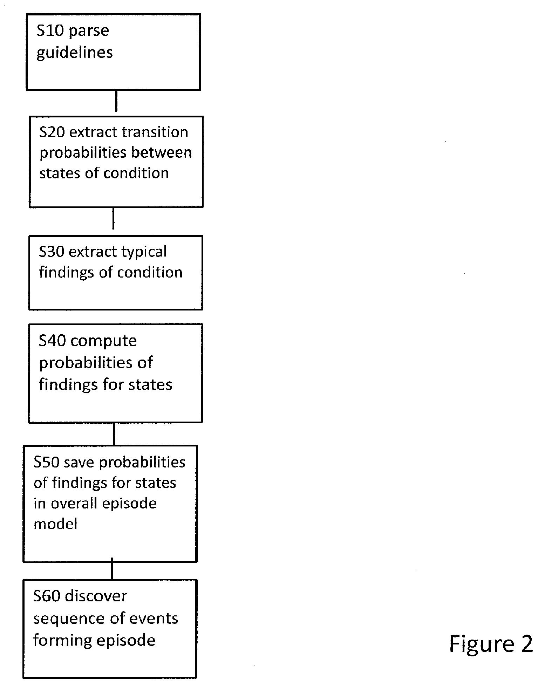

Figure 2 is a flowchart of a method embodiment. The method comprises steps S10 to S60. In step S10 published clinical guidelines are parsed. Step S20 extracts transition probabilities between the states. Step S30 extracts typical findings of the medical condition from domain knowledge as a finding model. Step S40 computes the probability of a particular finding for a particular state and step S50 saved the probabilities in an overall episode model including the state transition model and the finding model. Step S60 uses the overall episode model, and the set of medical records to discover a sequence of events that can be grouped into an episode.

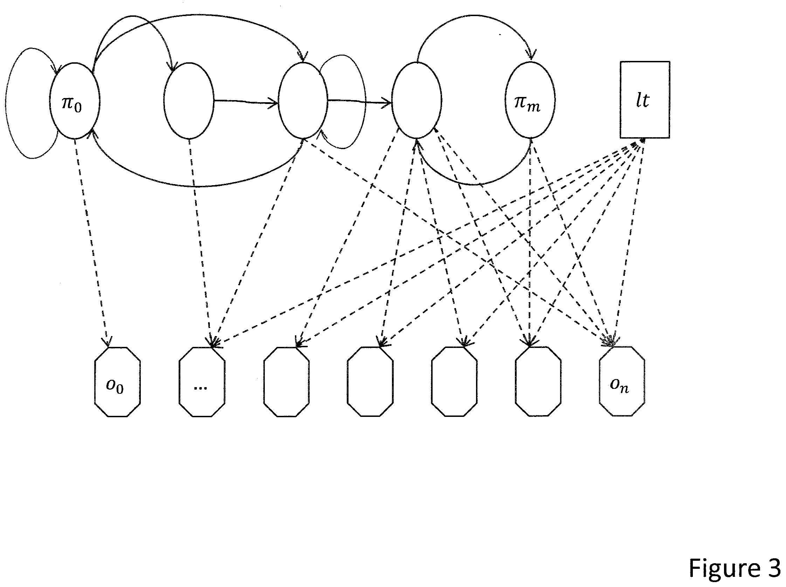

Figure 3 is a diagram of a generic episode model, constructed from a disease state transition model as the upper half of the diagram (excluding the leak term) and a finding model as the lower half, including links to the upper half to show the probabilities of particular findings for each state. For ease of representation, the associated probabilities are not shown.

The disease model in the top half is a static model defined or constructed from guidelines. The finding model in the bottom half (and links to the top half) only refers to the static knowledge of medical findings and other interactions between patients and medical system.

Circles represent different states of the given disease, octagons are observations/findings and the square is a leak term. $\pi$ is used to represent is a state or a stage of a disease.

The diagram includes the following components:

**States** $\pi_0^d, \dots, \pi_m^d$ of a disease $d$ are time points on the progression trajectory of a disease. Such states, for example, have significant and actionable meanings in the detection, diagnosis and treatment of the disease.

**Observations** $o_0^d, \dots, o_n^d$ of disease d with or without its comorbidities are the medical findings that can be documented or reported in health records. This includes the symptoms and findings identified by the medical professionals and complaints from the patients. Medical findings are defined in the finding model as probabilities over the given transition states of a disease.

**Leak term** is to accommodate the situation when a medical finding is observed while none of the transition states can be attributed to that finding. In other words, this is to cover unknown situations that are not transparent to (explained by) the above model.

The idea in this embodiment is that with such a model, it is possible to quantitatively define:

1. The transition of state that reflects the progression trajectory of diseases
2. The probability of state transition
3. The probability that, given a state of the disease, a medical finding can be observed.

With the above parameters known for major diseases, it is possible to fit a patient's medical record against such a model and identify the most appropriate sequence of patient's treatments or other findings (documented in his/her medical records) that can be grouped into one episode characterised by $E(d) = \pi_0^d, \dots, \pi_m^d$ where $d \in D$ is a disease and $D$ is the set of diseases whose progression trajectories are known or can be learnt from existing data. This gives benefit of fully understanding the episode (then one can group treatments together for analysis/ diagnosis/payment management). It may allow exclusion of co-morbidities.

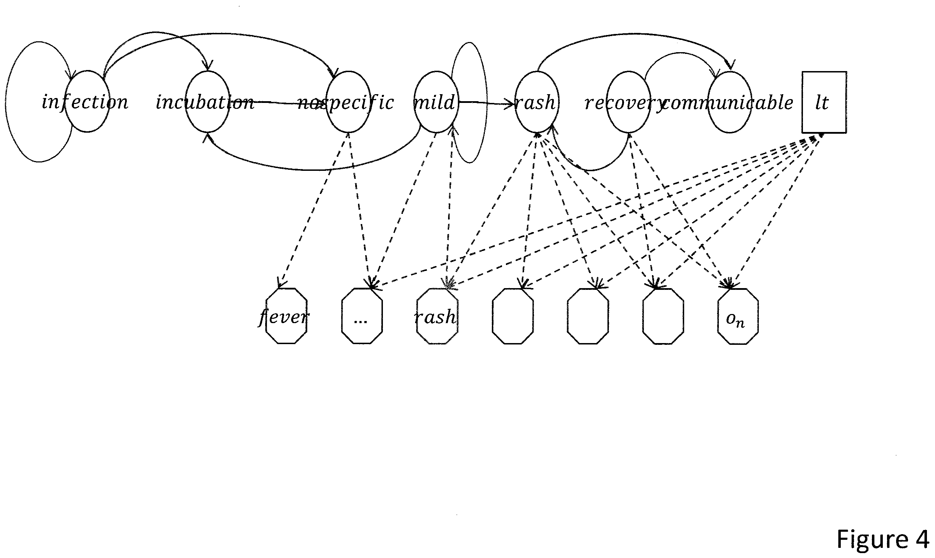

Figure 4 is a specific transition model of a generic episode. This transition model is also referred to as a directed graph. In most cases it should be acyclic, but in this case, we allow cycles, i.e. patients remain in the same state even after some interactions (treatments or medications). This is for better modelling of disease transitions. The states included in figure 4 are infection, incubation, no-specific, mild, rash, recovery, and communicable. A few observations are included as fever and rash.

**Learning progression trajectory**

It is important to first model the state transition model of a disease. This is done by mining data from the following sources:

1. Published medical protocols and guidelines. Standard, off-the-shelf process mining algorithms can be applied. The input of process mining is text or semi-completed process models; the output is a directed graph with cycles where vertices are key symptoms, key treatments/medications, and onset of comorbidities that defines the progression of a disease. Edges of the graph are directed defining how the states change.

2. Experts. The process model can be reviewed by experts when necessary.

Because the system aims to model an episode of a disease, not all information presented in medical guidelines are used. In the context of episode detection, useful information includes medications and treatments, comorbidities, test and examinations, and major symptoms and complaints. In other words, it covers the chargeable services provided by the medical professionals.

The state transition probability can be represented as a transit probability matrix as shown below. The sum of each row should be 1 to satisfy the probability requirement. It means that there is a probability of $s_{1j}$ for the disease to transit from stage 1 to stage j.

$$p_{11} \quad \dots, p_{1j}, \dots \quad p_{1n}$$

$$p_{i1} \quad \dots, p_{ij}, \dots \quad p_{in}$$

$$p_{n1} \quad \dots, p_{nj}, \dots \quad p_{nn}$$

In one example with rows and columns corresponding to the states above, cell values could be $p_{11}$ = 0.2 (20 percent of infected people will remain in the first stage without observable symptoms), $p_{12}$ = 0.6 (60 percent of infected patients reach the incubation state) and $p_{13}$ = 0.2 (20 percent of infected people show some non-specific symptoms).

The transition probability may be computed based on the following two approaches:

1. Ideally, when learning the process, the probability of transiting from one process step to another step can be computed using a statistical method. In practice, this can be computed based on a set of existing medical records.

2. When a complete transition probability cannot be learned or obtained from a statistical process or from domain experts, other data/knowledge source can be used.

For example, one solution can be achieved by leveraging an Internet-Search-Engine-based probability: if $\pi$ is connected with states (stages) $\pi_0$, ..., $\pi_i$ and not connected with states (stages) $\pi_j$, ..., $\pi_k$, the probability of $\pi$ transitting into $\pi_0$ is computed as Equation 1:

$$p(\pi_0|\pi) = \frac{p(\pi_0, \ \pi)}{p(\pi)} = \frac{hit(\pi, \pi_0) - \sum_{x=j}^{k} hit(\pi, \pi_0, \pi_x)}{\sum_{y=0}^{i} \left( hit(\pi, \pi_y) - \sum_{x=j}^{k} hit(\pi, \pi_0, \pi_x) \right)}$$

This computes the conditional probability of $\pi_0$ given $\pi$. The right hand side reads as: the conditional probability is the ratio of co-occurrence of $\pi$ and $\pi_0$ (excluding those cases when other states also appear) and the sum of such unique co-occurrence between $\pi$ and other "transitable" state from $\pi$.

$hit(\pi, \pi_0)$ refers in general to the co-occurrence of $\pi$ and $\pi_0$.
$p_{ij}$ in the above matrix can be computed accordingly pair-wise.

When obtaining such a public data based probability, it is desirable to focus the search space on more specific data sources or document corpus. For instance, the search can be confined to only medical publication domains such as PubMed (a service of the US National Library of Medicine® that: Provides free access to MEDLINE®, the NLM® database of indexed citations and abstracts to medical, nursing, dental, veterinary, health care, and preclinical sciences journal articles. Includes additional selected life sciences journals not in MEDLINE.)

In the transit probability matrix, values of one row should be scaled (if the value is obtained from different sources) and normalised so that the sum equals 1 to ensure proper probability characteristics.

**Obtaining the probability model of medical findings**

The relationship between disease states and observations is modelled with a bi-partite noisy-or Bayesian network (a directed acyclic graph in which variables are linked by edges including probabilities: the graph can be used to derive further probabilities). Here, both disease states and observations are present as binary variables. In this model, the states of diseases are marginally independent and given the value of disease states, the findings are conditionally independent. Both states and finding variables are binary, which indicates the presence (1) or absence (0) of a disease states and the positive (1) and negative (0) states of a finding.

A major assumption here is that the findings/observations only rely on the disease state rather than other "sibling" findings (which are also in the bottom half of the bi-partite model of figures 3 and 4). For instance, coughing as a symptom only relies on a stage of e.g. upper respiratory tract infection (URTI). It does not depend on high body temperature, which can be another symptom of URTI. This assumption simplifies the probabilistic model. There are cases that such a simplification cannot strictly apply (one symptom can lead to/have an effect on another symptom). But the benefit of the assumption is clear: overall computation is simplified.

The probability that given the state of a disease, observation $o_j$ can be obtained can be computed as $p(o_j|\Pi)$ in Equation 2 below: where $o_j$ is a binary variable and $f_{lt,j}$ signifies the failure probability of symptoms/findings given diseases. **The** failure probability is just the probability of false in a noisy-or gate (Bayes network), $f_{i,j}^{\pi_i} = 1 - p(o_j = T|\pi_i = T)$. It is the probability of a state does not trigger an observation. The overall model is a **leaky noisy-or network**, with the leak term for incomplete modelling where none of the state is true while the observation is still true/observed.

This equation accumulates an overall probability of observing o_j, given the status of states pi_0 to pi_m. The left part computes the probability when there is a positive observation (o_j = True or 1) while the right part computes the probability when there is a negative observation (o_j = False or 0). The left part basically says, when there is a positive observation, it is one minus the multiplication of all non-positive triggering (1 - $p(o_j = T|\pi_i = T)$). $\pi = \{\pi_i\}$ are related symptoms/findings.

$$p(o_j|\Pi) = \left(1 - f_{lt,j}\prod_{i=1}^{m} f_{i,j}^{\pi_i}\right)^{o_j} \left(f_{lt,j}\prod_{i=1}^{m} f_{i,j}^{\pi_i}\right)^{1-o_j}$$

o_j and 1-o_j is simply to switch on or switch off the left or right part of the equation and provides a uniform expression. This can also be achieved using conditions and multiple equations.

In the above equation, $f_{lt,j}$ and $f_{i,j}^{\pi_i}$ are computed as follows in Equation 3:

$$f_{lt,j} = p(o_j = 1|lt = 1)$$

$$f_{i,j}^{\pi_i} = \left(p(o_j = 1|\pi_i = 1)\right)^{\pi_i}$$

This equation gives a general way of using maximum likelihood to approximate the conditional probability. Hence $p(o_j|\Pi)$ is not literally speaking a probability. Other probability/statistical methods could be used as alternatives.

$p(o_j = 1|\pi_i = 1) = p(o_j = 1,\pi_i = 1)/p(\pi_i = 1)$ (the probability of a particular observation/finding being present for a state] can be acquired through one or more of the following methods, in the order specified or in any other combination:

1. If a training data set can be obtained, the probability should be learned from the training data set.
2. If a training data set is not available, public data should be used as indicated in the previous section.

   a. An initial co-occurrence is learnt using public data with the help of for instance general-purpose search engine.
   b. The co-occurrence values are normalised to ensure property probabilities are obtained.

The leak term can be approximated, for example, using the following method based on the literature using Equation 4:

$$f_{lt,j} = \frac{\prod_{i=0}^{m}\left(1 - p(\pi_i = 1) + p(\pi_i = 1) \cdot f_{i,j}\right)}{\prod_{i \in \{\exists \langle \pi_i, o_j \rangle\}}\left(1 - p(\pi_i = 1) + p(\pi_i = 1) \cdot f_{i,j}\right)}$$

Based on the methods in the above two subsections, models are constructed to be used by the episode grouper.

**Detecting an episode**

Episode detection is then reduced to finding the combination of observations against one patient that maximises the overall probability based on the state transition model and medical finding observation model which are linked in the overall (episode) model.

The overall model contains states and findings. A patient record contains events. These events are projected on the overall model so as to be grouped. The outcomes of the process is that such events are grouped into episode(s) for better understanding of the connections between the events in a patient record.

Basically, the system, based on the above two knowledge models (with corresponding numeric values), inputs an electronic patient record. It scans through the record and identifies the key events that can be aligned with those appear the corresponding knowledge models. It then computes the arrangement of states and findings that maximises the overall probability. This arrangement will then be output as a group of events that compose a complete episode.

**Events in medical records**

An "event" in an individual's medical record is an observable medical result or medical intervention that should have significant meaning in the course of diseases. Events can be extracted from medical guidelines as well as medical standards published by national and international authorities (e.g. WHO ICD, UMLS, etc.)

The definition of an event can also be extended with structured knowledge models, e.g. ontologies published by non-governmental organisations (e.g. Open Biomedical Ontologies).

In invention embodiments, a set of identifiable and distinguishable events is assumed to exist. This set of "events" can guide the process mining and knowledge extraction process.

**Calculation of probability**

Let "$E' = e_s, ..., e_t \subseteq O$ where O is the findings/observations". The elements in $E'$ observe the time sequence of $E''$ referring to the set of events in patient's record and the selected events that can be processed (that have correspondence in the finding model. In other words, elements selected do not need to be continuous but should observe the original order of time stamps associated with each event.

Given an **initial state** $\pi'$, the probability of observing $o_i$ is computed as $p(e_s, ... e_t | \pi, 0, \pi')$ in Equation 5:

$$p(e_s, ... e_t | \Pi, O, \pi') = \prod_{i=s}^{t} p(e_i | \Pi, O, \pi')$$

$$= \prod_{i=s}^{t} \left( \sum p(e_i \in O | \pi_j) p(\pi_j | \pi') + \sum p(e_i \in O | lt) + p(e_i \in O | \pi') \right)$$

The probability of have a particular sequence of events given the initial condition, the finding model and the state model is the multiplication of probabilities of all the individual events (assuming the events are not strongly depending on each other). This may be further broken down into the sum of probability of having a particular event given the initial state, having a particular event given the leak term and having a particular event based on the state transition probability. Here events are those from patient record but which may have correspondence in the finding model. Hence they are not named as findings as there are events which have no finding correspondence.

$e_i$ are findings instantiated in a particular patient's record. They are to be grouped based on the model. There may be $e_x$ that does not belong to any $O$ (findings) for the episode being investigated. $e_s, ... e_t$ are the events of a particular patient which is a subset of findings. The initial state $\pi'$ is the first documented / recorded interaction between an individual and the healthcare system. This could be the visit of the individual to a clinical centre or family doctor or the individual takes out a prescribed medicine.

For revisiting individuals, two approaches can be taken:

1. Strict approach: whether the revisit after a prolonged dormant period is a new episode or extension of the current episode, only the first point of contact will be treated as the initial state while all other visits (regardless of the length of dormant period) will be considered as a state transited from the initial period.

2. The above restriction increases the search space for find the optimal "fit". A relaxed approach may, therefore, be taken by leveraging domain heuristics. For instance, many existing models assume that when there is a dormant period over a threshold, for example longer than 6 months, a revisit/event due to the same complaints should be considered a new episode. With this relaxation, overall computation time can be reduced.

For all subsets of $E$, the probability of arriving such a sequence, based on the given initial state, state transition model and probability of observable medical findings, is calculated. This is equivalent to how much a sequence of observations (documented in a patient's medical records) is aligned with the theoretical observations based on the state transition of a disease and initial state of the patient. The sequence that presents the highest probability value is grouped together to form an episode (a course of the disease's progression in the context of that individual patient).

Let $\Phi = \{E'|E' \in 2^E \wedge |E'| > 1\}$ be the set of subsets of $E$ with size greater than 1. The process of episode detection is tantamount to finding the permutation of $\phi \in \Phi$ that maximises the probability. For instance, assume a patient has gone through {"X-ray", "drug A", "drug B", "Operation C"}. The powerset (set of all subsets including empty set and full set) of his/her events contains {{x-ray, A}, {x-ray, B}, {x-ray, C}, {A, B}, {A, C}, {B, C}, {x-ray, A, B}, {x-ray, A, C}, {x-ray, B, C}, {A, B, C}, {x-ray, A, B, C}}. The number of possible subsets excluding singleton and empty set are ($2^{|E|}$ - $|E|$ - 1). A full permutation of each subset is used to align with elements in $0$. The permutation that fits some elements in $0$ most (giving the highest probability) will be considered as from the same episode. In this example, assume the permutation $per(\{x\text{-ray}, A, C\}) = \{C, x\text{-ray}, A\}$ fit the $0^{D'} = \{D, C, x\text{-ray}, test1, test2, A, E\}$ with the greatest probability value. The patient is considered to have disease $D'$, where x-ray, A, C are grouped as one episode while B is excluded from this episode.

The complexity is likely to be as high as ($O^{2EO}$). In practice, it is possible to compute the probability of each permutation of each subset in parallel, so as to increase the overall system performance.

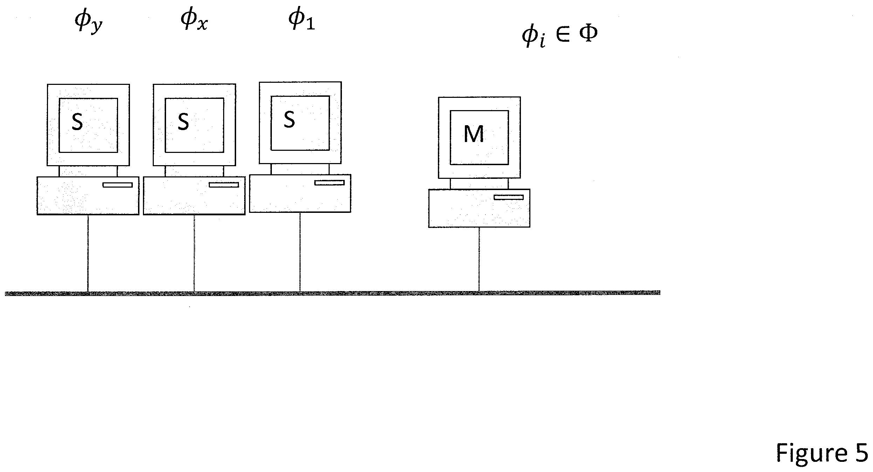

Figure 5 depicts the distribution of computations between a master machine and slave machines. Machine M is a master node which is responsible for randomly selecting a subset of patients' events and distributes these to other machines through the computer network. It is also responsible for collecting and aggregating results from different slave (S) machines. Each S machine has a copy of the episode grouping model (including state transition model and finding observation probability model). Upon receiving a subset $\phi_x$, S performs a full permutation of the elements in $\phi_x$ and for each permutation computes an overall fitness probability. S returns the maximum probability and the corresponding permutation back to M. M can then find the overall maximum probability amongst all the S machines: this is the subset of events that is defined as the episode.

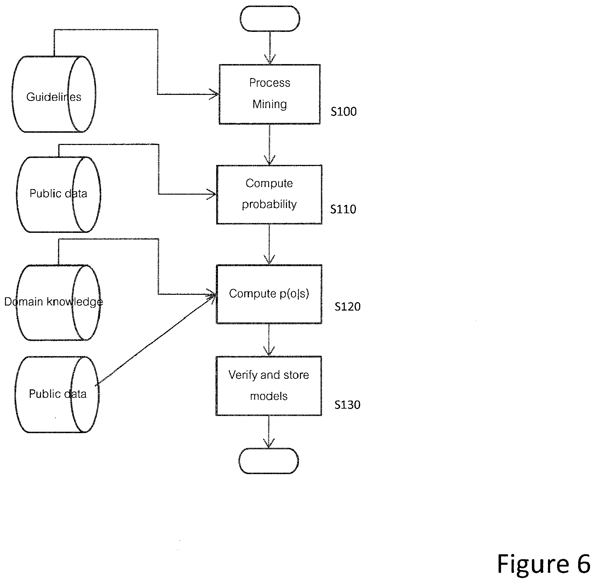

Figure 6 is a flow chart of model construction. The first flow chart depicts model construction. There is a process mining step S100 to acquire the state model using guidelines and/or other clinical pathway data.

Once the state model is learnt, the probability of transferring from one state to another state can be learnt or obtained from public data in step S110, potentially using the method proposed in Equation 1 used for deriving $p(\pi_0|\pi)$.

A refined state model can then be used to construct the overall model by computing the probability of observing certain findings based on domain knowledge (where the strict finding model comes from) and public data in step S120. The model may then be verified by domain experts and/or stored for further use in step S130.

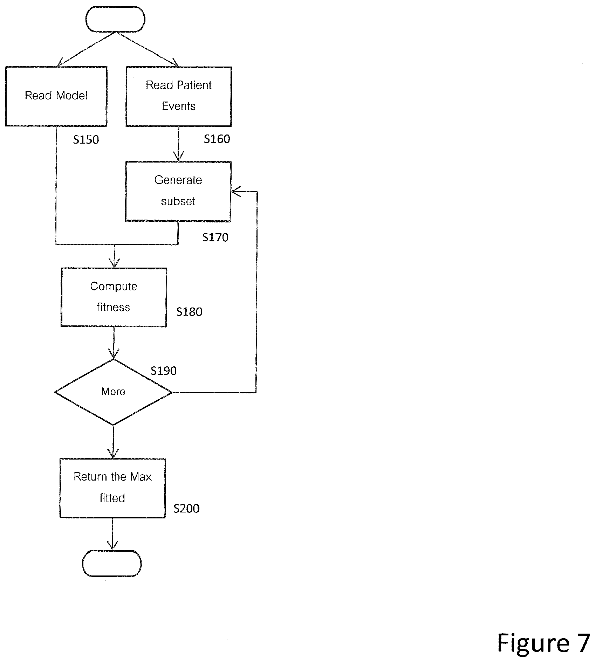

Figure 7 is a flow chart of episode discovery steps.

The system reads a saved model in S150; the system also reads a patient record (containing multiple events) in S160. It then randomly generates a subset of events out of the entire list of events from a patient's record in S170. This subset is then tested to compute fitness in S180 using Equation 6 to obtain an overall score of the given subset.

This subset-testing process continues until all subsets of the list of events have been tested (using "More" in step S190 and a loop back to subset generation). If there are no more subsets, the system returns the subset with the highest value computed using the Equation 5 as the suitable episode grouping of events.

The remaining events can be continuously processed, until all the events are grouped into an episode or until a threshold is reached. The threshold can be either a time limit or a number of remaining ungrouped events.

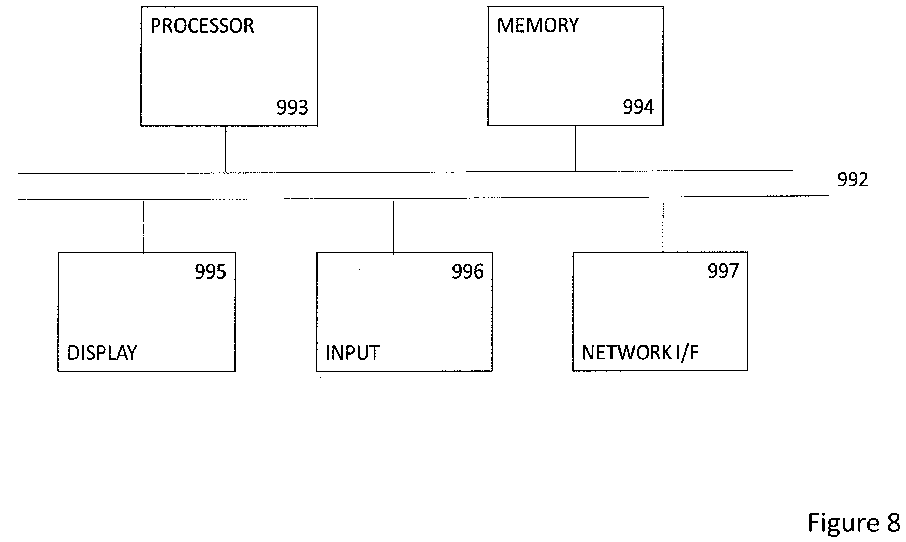

Figure 8 is a block diagram of a hardware computing device, such as a data storage server, which embodies the present invention, and which may be used to implement a method of an embodiment of the present invention to group patient events into episodes using the models as explained hereinbefore. The computing device comprises a processor 993, and memory, 994. Optionally, the computing device also includes a network interface 997 for communication with other computing devices, for example with other computing devices of invention embodiments. That is, the computing device may, for example, be a Master M machine as set out above.

For example, an embodiment may be composed of a network of such computing devices, one of which is the Master. Optionally, the computing device also includes one or more input mechanisms such as keyboard and mouse 996, and a display unit such as one or more monitors 995. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of the state transition leaner, clinical finding learner and episode grouper described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data (such as patient data, model data, subset of patient events or final grouping of events into an episode) stored by the computing device and may also display a cursor and dialog boxes and screens enabling interaction between a user and the programs and data stored on the computing device. The input mechanisms 996 may enable a user to input data and instructions to the computing device.

The network interface (network I/F) 997 may be connected to a network, such as the Internet to access the clinical guidelines, search engine and domain knowledge, and is connectable to other such computing devices via the network. The network I/F 997 may control data input/output from/to other apparatus via the network. Other peripheral devices such as microphone, speakers, printer, power supply unit, fan, case, scanner, trackerball etc. may be included in the computing device.

The state transition learner may comprise processing instructions stored on a portion of the memory 994, the processor 993 to execute the processing instructions, and a portion of the memory 994 to store states and probabilities of transition therebetween during the execution of the processing instructions. The output of the state transition learner in the form of a disease model/state transition graph may be stored on the memory 994 and/or on a connected storage unit, and may be transmitted to the clinical finding learner and/or the episode grouper.

The clinical finding learner may comprise processing instructions stored on a portion of the memory 994, the processor 993 to execute the processing instructions, and a portion of the memory 994 to store findings and probabilities of findings for given states during the execution of the processing instructions. The output of the clinical finding learner in the form of a finding model may be stored on the memory 994 and/or on a connected storage unit, and may be transmitted to the episode grouper.

The episode grouper may comprise processing instructions stored on a portion of the memory 994, the processor 993 to execute the processing instructions, and a portion of the memory 994 to store events and sequences and subsequences of events, along with probabilities of their fit to an episode. The output of the episode grouper in the form of a best-fit sub-sequence may be stored on the memory 994 and/or on a connected storage unit, and may be transmitted to the user interface.

Methods embodying the present invention may be carried out on a computing device such as that illustrated in Figure 8. Such a computing device need not have every component illustrated in Figure 8, and may be composed of a subset of those components. A method embodying the present invention may be carried out by a single computing device in communication with one or more data storage servers via a network. A method embodying the present invention may be carried out by a plurality of computing devices operating in cooperation with one another.

**Benefits**

The methods of invention embodiment can provide automated and unsupervised episode grouping that offers the following benefits:

1. Automatic detection and grouping of events in patients' medical records. This provides dual advantages:

   a. Medical findings can be grouped to offer better understandings of the courses of diseases and a coherent view for medical research purposes.
   b. Chargeable medical interactions can be grouped to provide clear basis for reimbursement and insurance compensation, and/or even to substantiate diagnosis

2. Automatic detection of events that are not part of a known disease episode. This helps to

   a. Identify atypical symptoms
   b. Highlight non-standard or non-conventional medical interactions for quality assurance purposes
   c. Monitor quality of health care services

3. Automatic computation of disease state transition probabilities and observable medical findings. This compliments conventional methods when data are not complete or not sufficient.

The system of invention embodiments can also contribute to the general clinical domain in the following aspects:

   1. It can help to differentiate apparently similar disease or co-morbidities by clearly defining progress and episode of each. This will help to give more accurate diagnosis and treatment.
   2. It can help to define and develop more personalised treatment as the progress of each disease with respect to a patient can be scoped.
   3. It can help to provide better health service at an early stage and project a patient's progress along established trajectories.

**Claims**

1. An apparatus for discovering a sequence of events in a set of medical records from a patient, the sequence forming an episode of a medical condition, the apparatus comprising:

   a state transition learner, to parse published clinical guidelines and to extract probabilities of transition between a number of states of a medical condition as a state transition model;
   a clinical finding learner, to extract typical findings of the medical condition from domain knowledge as a finding model and to compute the probability of a particular finding for a particular state and to save the probabilities in an overall episode model including the state transition model and the finding model; and
   an episode grouper, to use the overall episode model, and the set of medical records to discover a sequence of events, to group the sequence of events into an episode of the medical condition, and to differentiate the medical condition from apparently similar medical conditions or co-morbidities.

2. An apparatus according to claim 1, wherein the episode grouper is to discover a sequence as a subset of events in the set of medical record to be grouped into an episode, and to exclude remaining events as not a part of the medical condition.

3. An apparatus according to claim 1 or 2, wherein the state transition learner is to derive the probability of transition between states from internet search results, preferably from internet search results confined to one or more on-line medical publications.

4. An apparatus according to any preceding claim, wherein the clinical learning finder is to compute the probability of a particular finding for a particular state in the state transition model.

5. An apparatus according to claim 4, wherein the clinical learning finder uses a training data set to compute the probability of a particular finding for a particular state in the state transition model.

6. An apparatus according to any preceding claim, wherein the clinical learning finder is to compute the probability of a particular finding for a particular state using co-occurrence of the finding and the state in public data.

7. An apparatus according to any preceding claim, wherein the overall episode model contains, for each of one or more medical conditions, links between the findings in the finding model for the medical condition and the states in the state transition model for that medical condition and probabilities associated with the links.

8. An apparatus according to any preceding claim, wherein the overall episode model also contains links to a leak term and probabilities associated with the links, the leak term corresponding to a situation in which a finding is observed which is not relevant to any state in the state transition model for the medical condition.

9. An apparatus according to any preceding claim, wherein the episode grouper is to match sequences of events in the set of medical records to the overall episode model and to detect the best match between a sequence of events and the overall episode model.

10. An apparatus according to claim 9, wherein the episode grouper is to match the powerset of the sequence of events in the set of medical records to the overall episode model, with the exception of the empty set and/or any sets including a number of events below a threshold.

11. An apparatus according to claim 9 or 10, wherein the episode grouper is to match sequences of events to the overall episode model by calculating the probability of arriving at the sequence of events in the set of medical records for each sequence, based on a given initial state in the patient medical records, the state transition model and the probability of observable medical findings in the overall episode model corresponding to the events in the sequence of events.

12. An apparatus according to any preceding claim, wherein the episode grouper is to process remaining events in the set of medical records once a sequence of events has been grouped into an episode, by matching sequences of remaining events to another overall episode model, to identify another condition on the basis of one or more co-morbidities which might otherwise have been categorised as part of the same condition.

13. A computer-implemented method for discovering a sequence of events in a set of medical records from a patient, the sequence forming an episode of a medical condition, the method comprising:

parsing published clinical guidelines and extracting probabilities of transition between a number of states of a medical condition as a state transition model;
extracting typical findings of the medical condition from domain knowledge as a finding model;
computing the probability of a particular finding for a particular state;
saving the probabilities in an overall episode model including the state transition model and the finding model; and
using the overall episode model, and the set of medical records to discover a sequence of events, to group the sequence of events into an episode of the medical condition, and to differentiate the medical condition from apparently similar medical conditions or co-morbidities.

14. A computer program which when executed on a computer apparatus carries out a method for discovering a sequence of events in a set of medical records from a patient, the sequence forming an episode of a medical condition, the method comprising:

parsing published clinical guidelines and extracting probabilities of transition between a number of states of a medical condition as a state transition model;
extracting typical findings of the medical condition from domain knowledge as a finding model;
computing the probability of a particular finding for a particular state;
saving the probabilities in an overall episode model including the state transition model and the finding model; and
using the overall episode model, and the set of medical records to discover a sequence of events, to group the sequence of events into an episode of the medical condition, and to differentiate the medical condition from apparently similar medical conditions or co-morbidities.

Figure 1

S10 parse
guidelines

S20 extract transition
probabilities between
states of condition

S30 extract typical
findings of condition

S40 compute
probabilities of
findings for states

S50 save probabilities
of findings for states
in overall episode
model

S60 discover
sequence of events
forming episode

Figure 2

Figure 3

Figure 4

$\phi_y$     $\phi_x$     $\phi_1$          $\phi_i \in \Phi$

Figure 5

Figure 6

Figure 7

```
┌─────────────────┐          ┌─────────────────┐
│ PROCESSOR       │          │ MEMORY          │
│                 │          │                 │
│                 │          │                 │
│            993  │          │            994  │
└────────┬────────┘          └────────┬────────┘
         │                            │
─────────┼────────────────────────────┼──────────────── 992
         │              │             │
    ┌────┴────┐    ┌─────┴───┐    ┌────┴─────┐
    │    995  │    │    996  │    │    997   │
    │         │    │         │    │          │
    │         │    │         │    │          │
    │ DISPLAY │    │ INPUT   │    │NETWORK I/F│
    └─────────┘    └─────────┘    └──────────┘
```

Figure 8

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/265253 A1 (RAO R B [US] ET AL) 23 November 2006 (2006-11-23) * paragraphs [0022] - [0023] * * paragraphs [0039] - [0044] * * figure 3 * ----- | 1-14 | INV. G06F19/00 |
| A | US 8 732 096 B1 (GLUKHOV VACSLAV [GB]) 20 May 2014 (2014-05-20) * column 5, line 45 - column 7, line 35 * ----- | 1-14 | |

TECHNICAL FIELDS
SEARCHED (IPC)

G06F

The present search report has been drawn up for all claims

| Place of search | Date of completion of the search | Examiner |
|---|---|---|
| Munich | 13 July 2017 | Reinbold, Bernhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 16 6977

13-07-2017

| Patent document cited in search report | Publication date | Patent family member(s) | Publication date |
|---|---|---|---|
| US 2006265253 A1 | 23-11-2006 | US 2006265253 A1<br>WO 2006125145 A2 | 23-11-2006<br>23-11-2006 |
| US 8732096 B1 | 20-05-2014 | NONE | |

EPO FORM P0459

For more details about this annex : see Official Journal of the European Patent Office, No. 12/82